(19) Europäisches Patentamt
European Patent Office
Office européen des brevets

(11) **EP 4 769 307 A1**

(12) **EUROPEAN PATENT APPLICATION**
published in accordance with Art. 153(4) EPC

(43) Date of publication:
**01.07.2026 Bulletin 2026/27**

(21) Application number: **23952835.9**

(22) Date of filing: **30.10.2023**

(51) International Patent Classification (IPC):
*G06T 7/30* (2017.01)

(52) Cooperative Patent Classification (CPC):
**A61B 34/10; A61B 34/20; G06T 7/30**

(86) International application number:
**PCT/CN2023/127534**

(87) International publication number:
**WO 2025/060188 (27.03.2025 Gazette 2025/13)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **20.09.2023 CN 202311213577**

(71) Applicant: **Shanghai Taoimage Medical Tech. Co.,
Ltd.
Shanghai 200135 (CN)**

(72) Inventor: **WANG, Cong
Shanghai 201204 (CN)**

(74) Representative: **Argyma
14 Boulevard de Strasbourg
31000 Toulouse (FR)**

(54) **JOINT POSITIONING-BASED 2D-3D REGISTRATION METHOD AND SYSTEM**

(57) The present invention relates to technical field of 2D-3D registration, it proposes a 2D-3D registration method based on joint localization, comprising: S1: obtaining a coordinate transformation matrix between the biplane X-ray system and the joint localization device; S2: capturing anteroposterior/lateral X-ray images by using the biplane X-ray system, and recording a first spatial position and a first gravity direction of the joint center using the joint localization device; S3: establishing a coordinate system with the joint center of the 3D bone joint model as the coordinate origin, and determining a second gravity direction; converting the first spatial position and the first gravity direction recorded by the joint localization device into the coordinate system of the biplane X-ray system via the coordinate transformation relationship; S4: matching the coordinate origin and the second gravity direction of the 3D bone joint model with the first spatial position and the first gravity direction to complete coarse 2D-3D registration; S5: taking the result of the coarse registration as an initial value for fine registration based on iterative optimization to complete fine 2D-3D registration. This method improves the convergence, accuracy, and efficiency of the registration.

performing position calibration on a biplane X-ray system and a joint localization device to obtain a coordinate transformation matrix between coordinate systems of the biplane X-ray system and the joint localization device — S1

for each time point of patient's motion, synchronously capturing anteroposterior/lateral X-ray images of the patient's bone joint during patient's motion using the biplane X-ray system, and recording a first spatial position and a first gravity direction of the joint center using the joint localization device — S2

acquiring a threedimensional (3D) bone joint model, adjusting the 3D bone joint model to a position and pose consistent with the current position and pose of the patient's motion at each time point during patient's motion, establishing a coordinate system with the joint center of the 3D bone joint model as the coordinate origin, and determining a second gravity direction of the 3D bone joint model; meanwhile, converting the first spatial position and the first gravity direction recorded by the joint localization device at the corresponding time point of each frame of the anteroposterior/lateral X-ray images into the coordinate system of the biplane X-ray system via the coordinate transformation matrix — S3

matching the coordinate origin and the second gravity direction of the 3D bone joint model with the first spatial position and the first gravity direction to complete coarse 2D-3D registration — S4

taking the result of the coarse registration as an initial value for fine registration based on iterative optimization to complete fine 2D-3D registration — S5

FIG. 1

EP 4 769 307 A1

**Description**

**TECHNICAL FIELD**

The present invention relates to the technical field of two-dimensional (2D) - three-dimensional (3D) registration, and more particularly to a method and system for 2D-3D registration based on joint localization.

**BACKGROUND ART**

Image registration is widely used in image-guided interventions to simplify interventions, reduce invasiveness, and improve effectiveness. It has been extensively applied in radiation treatment planning, treatment verification, radiosurgery (e.g., arteriovenous malformations, endovascular stenting), interventional neuroradiology (minimally invasive surgeries of the head, neck, and spine), and vascular interventional radiology (angioplasty, shunting, embolization).

In orthopedic field, 2D-3D image registration is employed in spinal surgery, total hip arthroplasty, total knee arthroplasty, orthopedic diagnosis, and kinematic analysis. For kinematic analysis, 2D-3D registration between a 3D model of a joint and fluoroscopic video sequences acquired during various daily activities is utilized to kinematics analysis of both native and prosthetic knee and hip joints. The accuracy of this 2D-3D registration directly affects the precision of in vivo kinematic analysis for the joint. During bone joint kinematic analysis, a registration accuracy of approximately 1 millimeter and 1 degree is required.

Currently, conventional 2D-3D registration methods are based on simulated X-ray projection images, known as Digitally Reconstructed Radiographs (DRRs). A 3D dataset (or features extracted from it) is projected onto a fluoroscopic image plane at a specific position in 3D space to generate a DRR. The generated DRR is then compared with the real projection image of the 2D dataset according to a specific similarity metric. Registration involves an optimization procedure that iteratively searches for the 3D pose where the simulated image and real image exhibit the highest similarity. Upon convergence of the optimization procedure, the resulting 3D pose represents the optimal registration between the two datasets-i.e., the position of the 3D dataset within the 3D space of the imaging system and corresponds to the real projection image.

With the advancement of deep learning, researchers have recognized the potential to improve the efficiency of image registration. Miao (Miao S, Wang Z J, Liao R. A CNN Regression Approach for Real-Time 2D/3D Registration[J/OL]. IEEE Transactions on Medical Imaging, 2016, 35(5): 1352-1363. DOI:10.1109/TMI.2016.2521800) et al. proposed using a classical CNN model to extract feature regressing target position, then perform iteration to obtain accurate information. To reduce the number of iterations, Gao (Generalizing Spatial Transformers to Projective Geometry with Applications to 2D/3D Registration SpringerLink [EB/OL].[2023-05-16] https://link.springer.com/chapter/10.1007/978-3-030-59716-0_32) et al. proposed a spatial transformer network that enables differentiability between similarity and spatial position, significantly reducing the number of iterations. More recently, Liao (Liao H, Lin W A, Zhang J, et al. Multiview 2D/3D Rigid Registration via a Point-Of-Interest Network for Tracking and Triangulation[C/OL]//2019 IEEE/CVF Conference on Computer Vision and Pattern Recognition (CVPR). Long Beach, CA, USA: IEEE, 2019: 12630-12639 [2023-03-30].https://ieeexplore.ieee.org/document/8954218/.DOI:10.1109/CVPR.2019.01292.) proposed the POINT2 network, which detects points of interest in fluoroscopic images and directly localizes them through triangulation-eliminating the need for iterative optimization and greatly improving efficiency. Wang proposed a feature-based transfer learning method to extract features from fluoroscopic images, reducing requirement to real X-ray image while improving both the success rate and accuracy of registration.

However, conventional 2D-3D registration algorithms suffer from the non-convexity of the objective function, making it difficult for optimization operator to find the global optimal position. These 2D-3D registration algorithms are prone to get stuck in a local solution and highly dependent on the initial position and pose, which is often difficult to determine accurately. Moreover, randomly generated initial position and pose may differ significantly from the real position and pose, resulting in a large search space and requiring prolonged iterative optimization to achieve high-accuracy registration. Deep learning-based 2D-3D registration algorithms require a large amount of clinical image for training, yet still face limitations in terms of low registration accuracy and poor generalization ability. Additionally, the quality of images has a significant influence on the registration accuracy.

**SUMMARY**

In view of the above problems, an object of the present invention is to provide a 2D-3D registration method and system based on joint localization, so as to improve the convergence, accuracy and efficiency of registration.

The above object of the present invention is achieved by the following technical solutions:

A 2D-3D registration method based on joint localization, comprising:

S1: performing position calibration on a biplane X-ray system and a joint localization device to obtain a coordinate transformation matrix between coordinate systems of the biplane X-ray system and the joint localization device;

S2: for each time point of patient's motion, synchronously capturing anteroposterior/lateral X-ray images of the patient's bone joint during patient's motion using the biplane X-ray system, and recording a first spatial position and a first gravity direction of the joint center using the joint localization device;

S3: acquiring a three-dimensional (3D) bone joint model, adjusting the 3D bone joint model to a position and pose consistent with the current position and pose of the patient's motion at each time point during patient's motion, establishing a coordinate system with the joint center of the 3D bone joint model as the coordinate origin, and determining a second gravity direction of the 3D bone joint model; meanwhile, converting the first spatial position and the first gravity direction recorded by the joint localization device at the corresponding time point of each frame of the anteroposterior/lateral X-ray images into the coordinate system of the biplane X-ray system via the coordinate transformation matrix;

S4: matching the coordinate origin and the second gravity direction of the 3D bone joint model with the first spatial position and the first gravity direction to complete coarse 2D-3D registration;

S5: taking the result of the coarse registration as an initial value for fine registration based on iterative optimization to complete fine 2D-3D registration.

Further, the step of performing position calibration on the biplane X-ray system and the joint localization device to obtain the coordinate transformation matrix between the coordinate systems of the biplane X-ray system and the joint localization device comprising:

adjusting the biplane X-ray system to an initial position and fixing the joint localization device to ensure that the patient's joint is within the imaging region of both the biplane X-ray system and the joint localization device;

placing a calibration box in the fields of view of the biplane X-ray system and the joint localization device, wherein infrared reflective spheres with known spatial distribution are embedded on the surface of the calibration box, and metal steel balls are arranged at the centers of the infrared reflective spheres, the metal steel balls being concentric with the infrared reflective spheres;

capturing spatial coordinates of the centers of the metal steel balls using the biplane X-ray system, and capturing spatial coordinates of the centers of the infrared reflective spheres using the joint localization device;

performing spatial position calibration of the biplane X-ray system and the joint localization device based on the spatial coordinates of the centers of all the centers on the calibration box, to obtain the coordinate transformation matrix between the coordinate systems of the biplane X-ray system and the joint localization device.

Furthermore, the coarse 2D-3D registration and the fine 2D-3D registration are performed based on six-degree-of-freedom position and pose information derived from joint kinematics parameters, the six-degree-of-freedom position and pose information includes six parameters: (x, y, z, tx, ty, tz), wherein: x, y, z represent the coordinates of the joint center along the x-axis, y-axis, and z-axis, respectively; tx, ty, tz represent the rotation angles of the joint with respect to the x-axis, y-axis, and z-axis, respectively.

Furthermore, the step of matching the coordinate origin and the second gravity direction of the 3D bone joint model with the first spatial position and the first gravity direction to complete coarse 2D-3D registration comprising:

registering the coordinate origin of the 3D bone joint model with the first spatial position, thereby matching the three parameters (x, y, z) in the six-degree-of-freedom position and pose information;

registering the second gravity direction of the 3D bone joint model with the first gravity direction, thereby matching the tz parameter in the six-degree-of-freedom position and pose information.

Furthermore, the step of taking the result of the coarse registration as an initial value for fine registration based on iterative optimization to complete fine 2D-3D registration comprising:

projecting the 3D bone joint model onto the fluoroscopic Image plane via a ray-casting algorithm according to the six-degree-of-freedom position and pose information of the space in which the the 3D bone joint model lies, generating two virtual anteroposterior/lateral X-ray images with the same emitter and receiver position parameters as those of the biplane X-ray system when capturing the anteroposterior/lateral X-ray images;

after comparing the similarity between the virtual anteroposterior/lateral X-ray images and the anteroposterior/lateral X-ray images, optimizing the position and pose of the 3D bone joint model until the similarity between the virtual anteroposterior/lateral X-ray images and the anteroposterior/lateral X-ray images under the current position and pose of the 3D bone joint model reaches a predefined value, then completing match of parameters tx and ty in the six-degree-of-freedom position and pose information;

taking the six-degree-of-freedom position and pose information of the 3D bone joint model after fine 2D-3D registration as the result of fine 2D-3D registration based on iterative optimization.

Furthermore, optimizing the position and pose of the 3D bone joint model by utilizing a global optimizer after comparing the similarity between the virtual anteroposterior/lateral X-ray images and the anteroposterior/lateral X-ray images comprising:

employing comparison metrics including mutual information and normalization to compare the similarity between the virtual anteroposterior/lateral X-ray images and the anteroposterior/lateral X-ray images;
utilizing the global optimizer to optimize the position and pose of the 3D bone joint model by adopting evolutionary strategies with covariance matrix adaptation.

Furthermore, based on the fine 2D-3D registration results between the 3D bone joint model of the patient and the anteroposterior/lateral X-ray images at each time point during the patient' s motion, the relative positional relationships between joints of the patient when he is moving are obtained to analyze the patient' s joint motion function.

A 2D-3D registration system based on joint localization, comprising:

a device position calibration module configured to perform positional calibration on the biplanar X-ray system and the joint localization device, and obtain a coordinate transformation matrix between the coordinate systems of the biplanar X-ray system and the joint localization device;
a registration data acquisition module configured to synchronously capture, at each time point during the patient's motion, anteroposterior/lateral X-ray images of the patient's joints during motion using the biplanar X-ray system, and record, via the joint localization device, the first spatial position and the first gravity direction of the joint center;
a 3D model acquisition module configured to acquire a 3D bone joint model and adjust the 3D bone joint model to match the patient' s current position and pose at each time point of motion of the patient, establish a coordinate system with the joint center of the 3D bone joint model as the origin, determine the second gravity direction of the 3D bone joint model, and transform, using the coordinate transformation matrix, the first spatial position and the first gravity direction recorded by the joint localization device at the corresponding time point of each frame of the anteroposterior/lateral X-ray image into the coordinate system of the biplanar X-ray system;
a coarse registration module configured to match the coordinate origin and the second gravity direction of the 3D bone joint model with the first spatial position and the first gravity direction, thereby completing coarse 2D-3D registration;
a fine registration module configured to use the result of coarse registration as an initial value for iterative optimization-based fine registration, thereby completing fine 2D-3D registration.

A computer apparatus including a memory and one or more processors, wherein the memory stores computer code that, when executed by the one or more processors, causes the one or more processors to carry out the method described above.

A computer-readable storage medium storing computer code which, when executed, causes the method described above to be carried out.

Compared with the prior art, the present invention provides at least one of the following beneficial effects:

(1)The present invention transforms the coordinate system of the joint localization device into that of the biplanar X-ray system, enabling rapid acquisition of the relative positional relationship between the joint center and the X-ray emitter during the joint motion, obtaining an appropriate initial position and pose for the 2D-3D registration process as coarse registration result and using this coarse registration result as the initial value for iterative optimization-based fine registration, thereby expanding the capture range of the optimization operator, improving the convergence, accuracy, and efficiency of the registration.
(2)The present invention obtains the relative positional relationships between joints throughout the patient' s motion based on the fine 2D-3D registration results between the 3D bone joint model and the anteroposterior/lateral X-ray images for each frame, thus analyzing the joint motion function of the patient. This above technical solution enables rapid analysis of joint kinematic parameters for each frame based on the fine 2D-3D registration result, thereby facilitating fast analysis of the patient's joint motion function based on the kinematic parameters.

**BRIEF DESCRIPTION OF THE DRAWINGS**

FIG.1 is a overall flowchart of the joint localization based 2D-3D registration method according to the present invention;
FIG.2 is a detailed flowchart of the joint localization based 2D-3D registration method according to the present invention;
FIG.3 is a hardware architecture diagram of the joint localization-based 2D-3D registration method according to the present invention;
FIG.4 is a overall structural diagram of the joint localization-based 2D-3D registration method according to the present invention.

**DETAILED DESCRIPTION OF THE EMBODIMENTS**

To make the objectives, technical solutions, and advantages of the embodiments of the present application more clear, the technical solutions of the embodiments of the present application will be described in detail below with reference to the accompanying drawings. It is apparent that the described embodiments are merely a part of the embodiments of the present application, rather than all possible embodiments of the present application. Based on the embodiments disclosed herein, all embodiments conceivable by those skilled in the art without making inventive efforts shall fall within the scope of protection of the present application.

Those skilled in the art will understand that unless specifically stated otherwise, the singular forms "a," "an," "the," and "said" used herein may also include the plural forms. It should further be understood that the term "comprising" as used in the present disclosure means the presence of stated features, integers, steps, operations, elements, and/or components, but does not exclude the presence or addition of one or more other features, integers, steps, operations, elements, components, or groups thereof.

## FIRST EMBODIMENT

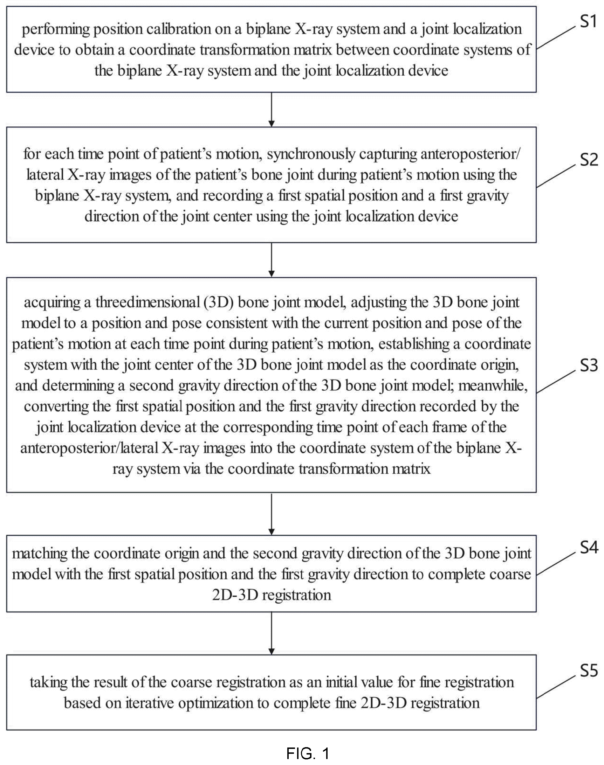

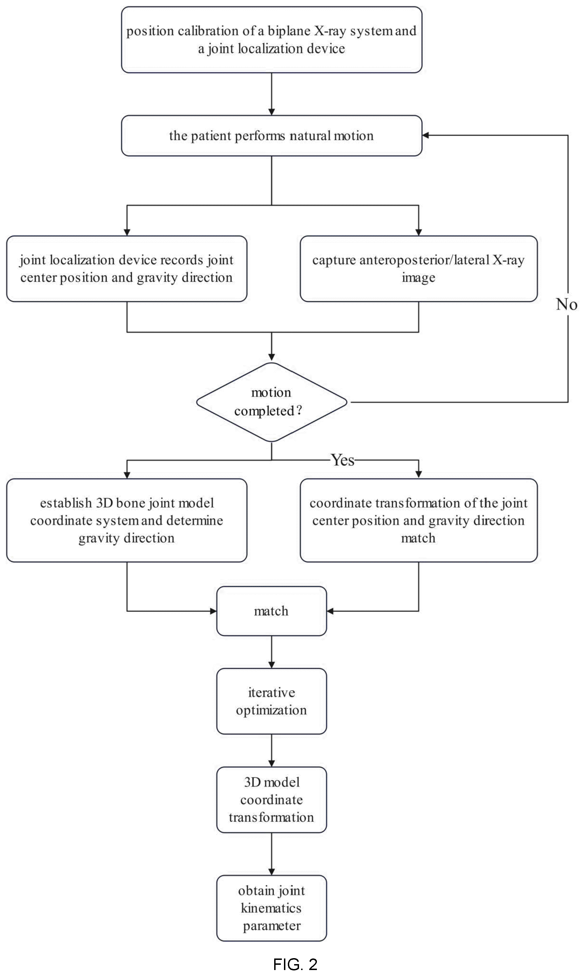

As shown in Figures 1 and 2, the present embodiment provides a joint localization-based 2D-3D registration method, which comprises the following steps: S1: performing position calibration on a biplane X-ray system and a joint localization device to obtain a coordinate transformation matrix between coordinate systems of the biplane X-ray system and the joint localization device.

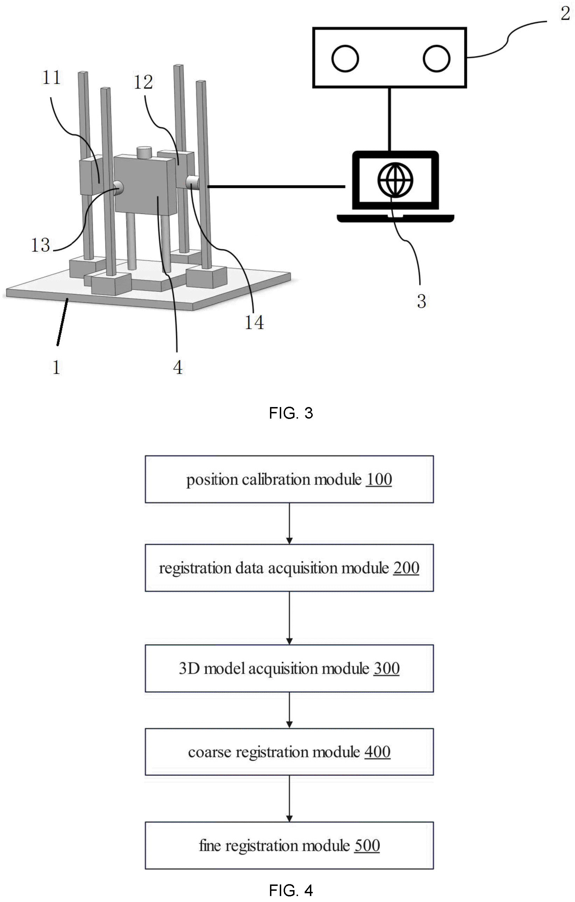

Specifically, in this embodiment, the hardware setup required for the joint localization-based 2D-3D registration method of the present invention is firstly established. As illustrated in FIG.3, the hardware configuration of the present invention includes a biplanar X-ray system 1, a joint localization device 2, and an host computer 3 that controls both the biplanar X-ray system 1 and the joint localization device 2 and receives data from them.

The biplanar X-ray system 1 comprises a first receiver 11, a second receiver 12, a first transmitter 14, and a second transmitter 13. The first receiver 11 and the first transmitter 14 are paired, the second receiver 12 and the second transmitter 13 are paired. The optical axes of the first transmitter 14 and the second transmitter 13 form an angle between 60 and 120 degrees. The first transmitter 14 and the second transmitter 13 respectively emit anteroposterior/lateral X-rays. The first receiver 11 and second receiver 12 receive the X-rays after they pass through the human body and generate corresponding X-ray images.

The reserved position 4, where human body motion occurs and where the calibration box mentioned later is placed, is located within the biplanar X-ray system 1. This reserved position 4 lies within the field of view of both the biplanar X-ray system 1 and the joint localization device 2. The joint localization device 2 may include, but is not limited to, Kinect, optoelectronic tracking device, or electromagnetic tracking device.

After the hardware configuration of the present invention are properly deployed, it is necessary to perform position calibration on the biplanar X-ray system 1 and the joint localization device 2 and obtain the coordinate transformation matrix between the coordinate systems of the biplanar X-ray system 1 and the joint localization device 2. This process specifically involves the following steps:

adjusting the biplanar X-ray system to its initial position and fix the joint localization device so that the patient's joint remains within the imaging area of both the biplanar X-ray system and the joint localization device;

placing a calibration box in the fields of view of the biplane X-ray system and the joint localization device, infrared reflective spheres with known spatial distribution are embedded on the surface of the calibration box, and metal steel balls are arranged at the centers of the infrared reflective spheres, the metal steel balls being concentric with the infrared reflective spheres;

capturing spatial coordinates of the centers of the metal steel balls using the biplane X-ray system, and capturing spatial coordinates of the centers of the infrared reflective spheres using the joint localization device;

performing spatial position calibration of the biplane X-ray system and the joint localization device based on the spatial coordinates of the centers of all the centers on the calibration box, to obtain the coordinate transformation matrix between the coordinate systems of the biplane X-ray system and the joint localization device.

The present invention imposes no specific limitations on the exact method used to obtain the coordinate transformation matrix between the coordinate system of the biplanar X-ray system and that of the joint localization device. For example, the method involves:

(1) obtaining the coordinates of all spherical centers on the calibration box in the coordinate system of the biplanar X-ray system, as well as their corresponding coordinates in the coordinate system of the joint localization device, and translate them into matrix form. Assuming there are N spherical centers, for these spherical centers, let X denote the matrix of coordinates in coordinate system corresponding to the biplanar X-ray system, Y denote the matrix of coordinates in the coordinate system of the joint localization device. X and Y are $3 \times N$ matrices, where each column represents the coordinate of one spherical center.

(2) normalizing the coordinate for each spherical center to eliminate the influence of scale factors. This can be achieved by subtracting coordinate average value from coordinate of each spherical center, and further dividing coordinate by its standard deviation.

(3) using the least squares method to solve for a 4×4 transformation matrix T such that Y=T*X. This is achieved by solving the following linear equations:

$$Y * X\textasciicircum T = T * X * X\textasciicircum T$$

where ^T denotes matrix transposition and * denotes matrix multiplication.

(4) extracting the coordinate transformation matrix from the solved T, which is generally a 3×3 submatrix.

S2: At each time point during the patient's motion, synchronously capturing anteroposterior and lateral X-ray images of the joint bones using the biplane X-ray system, and recording the first spatial position and the first gravity direction of the joint center using the joint localization device.

Specifically, when analyzing the joint motion function of the patient, the patient stands within the imaging area of both the biplane X-ray system and the joint localization device, and performs a series of daily movements. At every time point during the patient's motion, the joint localization device records the first spatial position and the gravity direction of the joint center and corresponds them, one to one, to the time point when the biplane X-ray system captures anteroposterior and lateral X-ray images.

S3: acquiring a 3D bone joint model, and at each time point during the patient's motion, adjusting the 3D bone joint model to make it have the same current position and pose as that of the patient's motion, establishing a coordinate system with the joint center of the 3D bone joint model as the origin of coordinate, determining the second gravity direction of the 3D bone joint model, and simultaneously transforming the first spatial position and the first gravity direction recorded by the joint localization device at the corresponding time point of each frame of anteroposterior and lateral X-ray images into the coordinate system of the biplane X-ray system using the coordinate transformation matrix.

Specifically, firstly it is need to acquire the 3D bone joint model, which can be dynamically and synchronously adjusted according to the position and pose relationship of each frame of anteroposterior and lateral X-ray images. The 3D bone joint model may take any form, including but not limited to 3D reconstruction from CT data or statistical shape models. During the registration process, the position and pose of the 3D bone joint model must be synchronized with the position and pose of each frame of anteroposterior and lateral X-ray images. A coordinate system is established with the joint center of the 3D bone joint model as the origin, and the second gravity direction is determined based on anatomical features of the joint. Meanwhile, the first spatial position and the first gravity direction recorded by the joint localization device at the time point corresponding to each frame of anteroposterior and lateral X-ray images are transformed into the coordinate system of the biplane X-ray system using the coordinate transformation relationship as obtained in Step S1.

The registration process, which includes coarse 2D-3D registration and fine 2D-3D registration, is a registration process based on six-degree-of-freedom (6DOF) position and pose information based on joint kinematic parameters. Specifically, the 6DOF position and pose information consists of six parameters (x, y, z, tx, ty, tz), where x, y, z denote the coordinates of the joint center along the x-axis, y-axis, and z-axis, respectively, and tx, ty, tz denote the rotation angles of the bone joint around the x-axis, y-axis, and z-axis, respectively. According to these six parameters in the 6DOF position and pose information, the 3D bone joint model is transformed in coordinate so that it corresponds to the anteroposterior and lateral X-ray images in space. The detailed registration process will be described in Steps S4 and S5.

S4: matching the coordinate origin and the second gravity direction of the 3D bone joint model with the first spatial position and the first gravity direction, respectively, to complete coarse 2D-3D registration.

In this embodiment, coarse 2D-3D registration specifically includes: registering the coordinate origin of the 3D bone joint model with the first spatial position to complete matching of the three parameters x, y, z in the 6DOF position and pose information; and registering the second gravity direction of the 3D bone joint model with the first gravity direction to completed matching of the parameter tz in the 6DOF position and pose information.

S5: using the result of the coarse registration as the initial value for the iterative optimization-based fine registration to complete fine 2D-3D registration.

In this embodiment, fine 2D-3D registration is specifically performed as follows:

projecting the 3D bone joint model onto the fluoroscopic image plane via a ray projection algorithm according to the six-degree-of-freedom position and pose information of the space in which the the 3D bone joint model lies, generating two virtual anteroposterior/lateral X-ray images with the same emitter and receiver position parameters as those of the biplane X-ray system when capturing the anteroposterior/lateral X-ray images.

Herein, projecting the 3D bone joint model onto the fluoroscopic image plane via a ray projection algorithm according to the six-degree-of-freedom position and pose information of the space in which the the 3D bone joint model lies, can be carried out by the following steps (provided as an example only, not intended to limit the present invention):

(1) defining fluoroscopic camera parameters: determining the parameters of the fluoroscopic camera, including camera position, viewing direction, focal length, etc. These parameters will influence the projection result.

(2) applying the 6DOF position and pose information to the 3D model: transforming the 3D model using the 6DOF position and pose information to convert it from the model coordinate system to the world coordinate system.

(3) performing ray projection: for each vertex of the 3D model, determining its projected position on the fluoroscopic image plane using the ray projection algorithm. The ray projection can be calculated by using the fluoroscopic camera parameters and the vertex positions of the 3D model.

(4) obtaining the fluoroscopic projection image: mapping the projected positions onto the fluoroscopic image plane to form the fluoroscopic projection image. Coordinate transformations and interpolation may be applied as needed to achieve a smoother image result.

after comparing the similarity between the virtual anteroposterior/lateral X-ray images and the anteroposterior/lateral X-ray images, optimizing the position and pose of the 3D bone joint model using a global optimizer until the similarity between the virtual anteroposterior/lateral X-ray images and the anteroposterior/lateral X-ray images under the current position and pose of the 3D bone joint model reaches a predefined value, then completing match of parameters tx and ty in the six-degree-of-freedom position and pose information.

employing comparison metrics including mutual information and normalization to compare the similarity between the virtual anteroposterior/lateral X-ray images and the anteroposterior/lateral X-ray images.

The similarity comparison is a method for quantifying the degree of similarity between two objects. In the comparison metrics, the mutual information and normalized correlation are commonly used.

(1) Mutual Information: Mutual information is a metric used to measure the correlation or dependency between two random variables. In similarity comparison, mutual information can be applied to evaluate the correlation between features or attributes of two objects. A higher mutual information indicates a greater similarity between the two objects.

(2) Normalized Correlation: Normalized correlation measures the similarity between two vectors by computing their correlation. Correlation is typically calculated using correlation coefficients, such as the Pearson correlation coefficient. Normalized correlation scales the correlation coefficient to the range [-1, 1], where 1 represents perfect positive correlation, -1 represents perfect negative correlation, and 0 indicates no correlation. The closer the normalized correlation approaches 1, the higher the similarity between the two objects.

In addition to mutual information and normalized correlation, other commonly used similarity metrics including cosine similarity, Euclidean distance, and Hamming distance, are used. The choice of an appropriate metric depends on the specific application scenario and the type of objects being compared.

utilizing the global optimizer to optimize the position and pose of the 3D bone joint model by adopting evolutionary strategies with covariance matrix adaptation.

Herein, the Covariance Matrix Adaptation Evolution Strategy (CMA-ES) is an evolutionary algorithm for solving optimization problems. It is a variant of Evolution Strategies (ES) that adaptively adjusts the covariance matrix to realize exploration and convergence of the search space.

The core idea of CMA-ES is to adaptively estimate and update a probability distribution during the evolutionary process, enabling more efficient search in the optimization space. Specifically, CMA-ES maintains a multivariate Gaussian distribution model, where the mean vector represents the current position of the solution, and the covariance matrix represents the search directions and step sizes during the search process.

The CMA-ES algorithm proceeds as follows:

(1) initialization: randomly generate an initial solution vector and the covariance matrix.

(2) generate individuals: generate a set of solution vectors as candidate solutions according to the current multivariate Gaussian distribution model.

(3) evaluate individuals: assess each generated candidate solution vector to compute fitness value.

(4) update model: update the model based on the fitness value using the covariance matrix. better solutions will influence the mean value and the covariance matrix of the model, enabling more effective exploration of the optimization space.

(5) convergence check: determine whether stopping criteria are met, such as the maximum number of iterations has been reached or the fitness is sufficiently high.

(5) iterate: if the stopping criteria are not satisfied, repeat steps (2) to (5) iteratively to continue searching the optimization space.

CMA-ES adaptively adjusts average value and covariance matrix to adapt to the characteristics of the problem, thereby achieving better search capability. It performs well in handling high-dimensional, nonlinear, and non-convex optimization problems and does not require gradient calculation of the objective function. In summary, the Evolution Strategy of Covariance Adaptive adjustment (CMA-ES) is an adaptive evolutionary algorithm that explores and optimizes within the search space by estimating and updating the covariance matrix. It demonstrates excellent performance and robustness in solving optimization problems.

The six-degree-of-freedom (6DOF) position and pose information of the 3D bone joint model obtained after performing the fine 2D-3D registration is taken as the result of the iterative optimization-based fine 2D-3D registration.

Furthermore, after completing the registration for each frame, the relative spatial relationships between joints during the patient's motion are determined based on the fine 2D-3D registration results of the 3D bone joint model and the anteroposterior/ lateral X-ray images at each time point during the patient's motion. These relationships are then used to analyze the joint motion function of the patient.

For example, taking the knee joint as an instance, through 2D-3D registration of each frame of anteroposterior/lateral X-ray images, six parameters of the six-degree-of-freedom (6DOF) positional information of the femur and tibia in space can be obtained respectively. Based on these six parameters, these six parameters of the femur and tibia can be transformed into the global coordinate system. By analyzing the relationship between anatomical sites of the femur and tibia, the relative movement between the tibia and femur for each frame and clinical parameter such as internal rotation and abduction can be determined to further analyze the joint motion function of the patient.

**SECOND EMBODIMENT**

As shown in FIG.4, the present embodiment provides a joint localization-baa position calibration module 100 configured to perform positional calibration on the biplanar X-ray system and the joint localization device, and obtain a coordinate transformation matrix between the coordinate systems of the biplanar X-ray system and the joint localization device;

> a position calibration module 100 configured to perform positional calibration on the biplanar X-ray system and the joint localization device, and obtain a coordinate transformation matrix between the coordinate systems of the biplanar X-ray system and the joint localization device;
> a registration data acquisition module 200 configured to synchronously capture, at each time point during the patient's motion, anteroposterior/lateral X-ray images of the patient's joints during motion using the biplanar X-ray system, and record, via the joint localization device, the first spatial position and the first gravity direction of the joint center;
> a 3D model acquisition module 300 configured to acquire a 3D bone joint model and adjust the 3D bone joint model to match the patient's current position and pose at each time point of motion of the patient, establish a coordinate system with the joint center of the 3D bone joint model as the origin, determine the second gravity direction of the 3D bone joint model, and transform, using the coordinate transformation matrix, the first spatial position and the first gravity direction recorded by the joint localization device at the corresponding time point of each frame of the anteroposterior/lateral X-ray image into the coordinate system of the biplanar X-ray system;

a coarse registration module 400 configured to match the coordinate origin and the second gravity direction of the 3D bone joint model with the first spatial position and the first gravity direction, thereby completing coarse 2D-3D registration;

a fine registration module 500 configured to use the result of coarse registration as an initial value for iterative optimization-based fine registration, thereby completing fine 2D-3D registration.

A computer-readable storage medium storing computer code which, when executed, causes the method described above to be carried out. It will be appreciated by those skilled in the art that all or part of the steps in the various methods of the above-described embodiments can be implemented by a program instructing relevant hardware. Such program may be stored in a computer-readable storage medium comprising read-only memory (ROM), random access memory (RAM), magnetic disks, optical discs, and the like.

The foregoing descriptions are merely preferred embodiments of the present invention, and the scope of protection of the present invention is not limited to the above embodiments. All technical solutions falling within the spirit and principles of the present invention shall fall within the scope of protection of the present invention. It should be noted that various improvements and modifications made by those skilled in the art without departing from the principles of the present invention shall also be regarded as within the protection scope of the present invention.

The technical features described in the above embodiments may be combined in any manner. For the sake of brevity, not all possible combinations of these technical features in these embodiments are explicitly described. However, as long as the combinations of these technical features are not contradictory, they should all be considered as fall within the scope as recorded by this Specification.

It should also be noted that the above embodiments may be freely combined according to requirements. The foregoing descriptions are merely preferred embodiments of the present invention. It should be pointed out that for those skilled in the art, various improvements and modifications may still be made without departing from the principles of the present invention, and such improvements and modifications shall likewise be regarded as falling within the protection scope of the present invention.sed 2D-3D registration system, comprising:

## Claims

1. A 2D-3D registration method based on joint localization, comprising:

    S1: performing position calibration on a biplane X-ray system and a joint localization device to obtain a coordinate transformation matrix between coordinate systems of the biplane X-ray system and the joint localization device;

    S2: for each time point of patient's motion, synchronously capturing anteroposterior/lateral X-ray images of the patient's bone joint during patient's motion using the biplane X-ray system, and recording a first spatial position and a first gravity direction of joint center using the joint localization device;

    S3: acquiring a three-dimensional (3D) bone joint model, adjusting the 3D bone joint model to a position and pose consistent with current position and pose of the patient's motion at each time point during patient's motion, establishing a coordinate system with the joint center of the 3D bone joint model as coordinate origin, and determining a second gravity direction of the 3D bone joint model; meanwhile, converting the first spatial position and the first gravity direction recorded by the joint localization device at the corresponding time point of each frame of the anteroposterior/lateral X-ray images into the coordinate system of the biplane X-ray system via the coordinate transformation matrix;

    S4: matching the coordinate origin and the second gravity direction of the 3D bone joint model with the first spatial position and the first gravity direction to complete coarse 2D-3D registration;

    S5: taking the result of the coarse registration as an initial value for fine registration based on iterative optimization to complete fine 2D-3D registration.

2. The 2D-3D registration method based on joint localization according to claim 1, wherein the step of performing position calibration on the biplane X-ray system and the joint localization device to obtain the coordinate transformation matrix between the coordinate systems of the biplane X-ray system and the joint localization device comprising:

    adjusting the biplane X-ray system to an initial position and fixing the joint localization device to ensure that the patient's joint is within the imaging region of both the biplane X-ray system and the joint localization device;

    placing a calibration box in the fields of view of the biplane X-ray system and the joint localization device, wherein infrared reflective spheres with known spatial distribution are embedded on the surface of the calibration box, and metal steel balls are arranged at the centers of the infrared reflective spheres, the metal steel balls being concentric with the infrared reflective spheres;

capturing spatial coordinates of the centers of the metal steel balls using the biplane X-ray system, and capturing spatial coordinates of the centers of the infrared reflective spheres using the joint localization device;

performing spatial position calibration of the biplane X-ray system and the joint localization device based on the spatial coordinates of the centers of all the centers on the calibration box, to obtain the coordinate transformation matrix between the coordinate systems of the biplane X-ray system and the joint localization device.

3. The 2D-3D registration method based on joint localization according to claim 1, wherein the coarse 2D-3D registration and the fine 2D-3D registration are performed based on six-degree-of-freedom position and pose information derived from joint kinematics parameters, the six-degree-of-freedom position and pose information includes six parameters: (x, y, z, tx, ty, tz), wherein: x, y, z represent the coordinates of the joint center along the x-axis, y-axis, and z-axis, respectively; tx, ty, tz represent the rotation angles of the joint with respect to the x-axis, y-axis, and z-axis, respectively.

4. The 2D-3D registration method based on joint localization according to claim 3, wherein the step of matching the coordinate origin and the second gravity direction of the 3D bone joint model with the first spatial position and the first gravity direction to complete coarse 2D-3D registration comprising:

    registering the coordinate origin of the 3D bone joint model with the first spatial position, thereby matching the three parameters x, y, z in the six-degree-of-freedom position and pose information;

    registering the second gravity direction of the 3D bone joint model with the first gravity direction, thereby matching the tz parameter in the six-degree-of-freedom position and pose information.

5. The 2D-3D registration method based on joint localization according to claim 3, wherein the step of taking the result of the coarse registration as an initial value for fine registration based on iterative optimization to complete fine 2D-3D registration comprising:

    projecting the 3D bone joint model onto the fluoroscopic image plane via a ray-projection algorithm according to the six-degree-of-freedom position and pose information of the space in which the the 3D bone joint model lies, generating two virtual anteroposterior/lateral X-ray images with the same emitter and receiver position parameters as those of the biplane X-ray system when capturing the anteroposterior/lateral X-ray images;

after comparing the similarity between the virtual anteroposterior/lateral X-ray images and the anteroposterior/lateral X-ray images, optimizing the position and pose of the 3D bone joint model until the similarity between the virtual anteroposterior/lateral X-ray images and the anteroposterior/lateral X-ray images under the current position and pose of the 3D bone joint model reaches a predefined value, then completing match of parameters tx and ty in the six-degree-of-freedom position and pose information; taking the six-degree-of-freedom position and pose information of the 3D bone joint model after fine 2D-3D registration as the result of fine 2D-3D registration based on iterative optimization.

6. The 2D-3D registration method based on joint localization according to claim 5, wherein optimizing the position and pose of the 3D bone joint model after comparing the similarity between the virtual anteroposterior/lateral X-ray images and the anteroposterior/lateral X-ray images comprising:

employing comparison metrics including mutual information and normalization to compare the similarity between the virtual anteroposterior/lateral X-ray images and the anteroposterior/lateral X-ray images;
utilizing a global optimizer to optimize the position and pose of the 3D bone joint model by adopting evolutionary strategies with covariance matrix adaptation.

7. The 2D-3D registration method based on joint localization according to claim 1, further comprising:
based on the fine 2D-3D registration results between the 3D bone joint model of the patient and the anteroposterior/lateral X-ray images at each time point during the patient's motion, obtaining the relative positional relationships between the joints of the patient when the patient is moving, to analyze the joint motion function of the patient.

8. A 2D-3D registration system based on joint localization, comprising:

a position calibration module configured to perform positional calibration on the biplanar X-ray system and the joint localization device, and obtain a coordinate transformation matrix between the coordinate systems of the biplanar X-ray system and the joint localization device;
a registration data acquisition module configured to synchronously capture, at each time point during the patient's motion, anteroposterior/lateral X-ray images of the patient's joints during motion using the biplanar X-ray system, and record, via the joint localization device, the first spatial position and the first gravity direction of the joint center;
a 3D model acquisition module configured to acquire a 3D bone joint model and adjust the 3D bone joint model to match the patient's current position and pose at each time point of motion of the patient, establish a coordinate system with the joint center of the 3D bone joint model as the origin, determine the second gravity direction of the 3D bone joint model, and transform, using the coordinate transformation matrix, the first spatial position and the first gravity direction recorded by the joint localization device at the corresponding time point of each frame of the anteroposterior/lateral X-ray image into the coordinate system of the biplanar X-ray system;
a coarse registration module configured to match the coordinate origin and the second gravity direction of the 3D bone joint model with the first spatial position and the first gravity direction, thereby completing coarse 2D-3D registration;
a fine registration module configured to use the result of coarse registration as an initial value for iterative optimization-based fine registration, thereby completing fine 2D-3D registration.

9. A computer apparatus including a memory and one or more processors, wherein the memory stores computer code that, when executed by the one or more processors, causes the one or more processors to carry out the method as described in any one of claims 1-7.

10. A computer-readable storage medium storing computer code which, when executed, causes the method as described in any one of claims 1-7 to be carried out.

performing position calibration on a biplane X-ray system and a joint localization device to obtain a coordinate transformation matrix between coordinate systems of the biplane X-ray system and the joint localization device — S1

for each time point of patient's motion, synchronously capturing anteroposterior/ lateral X-ray images of the patient's bone joint during patient's motion using the biplane X-ray system, and recording a first spatial position and a first gravity direction of the joint center using the joint localization device — S2

acquiring a threedimensional (3D) bone joint model, adjusting the 3D bone joint model to a position and pose consistent with the current position and pose of the patient's motion at each time point during patient's motion, establishing a coordinate system with the joint center of the 3D bone joint model as the coordinate origin, and determining a second gravity direction of the 3D bone joint model; meanwhile, converting the first spatial position and the first gravity direction recorded by the joint localization device at the corresponding time point of each frame of the anteroposterior/lateral X-ray images into the coordinate system of the biplane X-ray system via the coordinate transformation matrix — S3

matching the coordinate origin and the second gravity direction of the 3D bone joint model with the first spatial position and the first gravity direction to complete coarse 2D-3D registration — S4

taking the result of the coarse registration as an initial value for fine registration based on iterative optimization to complete fine 2D-3D registration — S5

FIG. 1

FIG. 2

FIG. 3

FIG. 4

## INTERNATIONAL SEARCH REPORT

| International application No. |
| --- |
| **PCT/CN2023/127534** |

| | |
| --- | --- |
| **A. CLASSIFICATION OF SUBJECT MATTER** | |
| G06T 7/30(2017.01)i | |
| According to International Patent Classification (IPC) or to both national classification and IPC | |

**B. FIELDS SEARCHED**

Minimum documentation searched (classification system followed by classification symbols)

IPC: G06T

Documentation searched other than minimum documentation to the extent that such documents are included in the fields searched

Electronic data base consulted during the international search (name of data base and, where practicable, search terms used)

CNTXT, VEN, DWPI, ENTXT: 上海涛影医疗科技有限公司, 王聪, 配准, 二维, 三维, 2D, 3D, 坐标, 矩阵, 双平面X光, 关节, 定位, 模型, 重力, 迭代, 收敛, 精度, registration, two-dimensional, three-dimensional, coordinates, matrix, X-ray, joint, localization, model, gravity, iteration, precision

**C. DOCUMENTS CONSIDERED TO BE RELEVANT**

| Category* | Citation of document, with indication, where appropriate, of the relevant passages | Relevant to claim No. |
| --- | --- | --- |
| A | CN 115281699 A (SHANGHAI TAOIMAGE MEDICAL TECHNOLOGY CO., LTD.) 04 November 2022 (2022-11-04)<br>description, paragraphs [0054]-[0103] | 1-10 |
| A | CN 103247056 A (FIRST AFFILIATED HOSPITAL OF THIRD MILITARY MEDICAL UNIVERSITY OF PLA) 14 August 2013 (2013-08-14)<br>entire document | 1-10 |
| A | CN 111973204 A (SHANGHAI JIAO TONG UNIVERSITY) 24 November 2020 (2020-11-24)<br>entire document | 1-10 |
| A | CN 112132876 A (TIANJIN UNIVERSITY) 25 December 2020 (2020-12-25)<br>entire document | 1-10 |
| A | CN 112184782 A (SHANGHAI JIAO TONG UNIVERSITY) 05 January 2021 (2021-01-05)<br>entire document | 1-10 |
| A | CN 115018977 A (JILIN UNIVERSITY) 06 September 2022 (2022-09-06)<br>entire document | 1-10 |

| ☑ Further documents are listed in the continuation of Box C. | ☑ See patent family annex. |
| --- | --- |

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **06 December 2023** | **12 December 2023** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| | International application No. |
|---|---|
| | **PCT/CN2023/127534** |

### C. DOCUMENTS CONSIDERED TO BE RELEVANT

| Category* | Citation of document, with indication, where appropriate, of the relevant passages | Relevant to claim No. |
|---|---|---|
| A | EP 1124201 A1 (BTG INTERNATIONAL LIMITED) 16 August 2001 (2001-08-16)<br>entire document | 1-10 |
| A | KR 102460864 B1 (DAEGU GYEONGBUK INSTITUTE OF SCIENCE AND TECHNOLOGY) 28 October 2022 (2022-10-28)<br>entire document | 1-10 |
| A | WO 2023089566 A1 (REMEX MEDICAL CORP. et al.) 25 May 2023 (2023-05-25)<br>entire document | 1-10 |

Form PCT/ISA/210 (second sheet) (July 2022)

**INTERNATIONAL SEARCH REPORT**
**Information on patent family members**

International application No.

**PCT/CN2023/127534**

| Patent document cited in search report | | | Publication date (day/month/year) | Patent family member(s) | | | Publication date (day/month/year) |
|---|---|---|---|---|---|---|---|
| CN | 115281699 | A | 04 November 2022 | None | | | |
| CN | 103247056 | A | 14 August 2013 | CN | 103247056 | B | 13 January 2016 |
| CN | 111973204 | A | 24 November 2020 | CN | 111973204 | B | 06 September 2022 |
| CN | 112132876 | A | 25 December 2020 | CN | 112132876 | B | 29 March 2022 |
| CN | 112184782 | A | 05 January 2021 | CN | 112184782 | B | 23 May 2023 |
| CN | 115018977 | A | 06 September 2022 | None | | | |
| EP | 1124201 | A1 | 16 August 2001 | None | | | |
| KR | 102460864 | B1 | 28 October 2022 | None | | | |
| WO | 2023089566 | A1 | 25 May 2023 | US | 2023149083 | A1 | 18 May 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MIAO S** ; **WANG Z J** ; **LIAO R.** A CNN Regression Approach for Real-Time 2D/3D Registration[J/OL. *IEEE Transactions on Medical Imaging*, 2016, vol. 35 (5), 1352-1363
- **GAO**. *Generalizing Spatial Transformers to Projective Geometry with Applications to 2D/3D Registration SpringerLink[EB/OL*, 16 May 2023, https://link. springer.com/chapter/10.1007/978-3-030-59716-0_32

- Multiview 2D/3D Rigid Registration via a Point-Of-Interest Network for Tracking and Triangulation [C/OL. **LIAO H** ; **LIN W A** ; **ZHANG J et al.** 2019 IEEE/CVF Conference on Computer Vision and Pattern Recognition (CVPR). IEEE, 2019